(19) Europäisches Patentamt
European Patent Office
Office européen des brevets

(11) Veröffentlichungsnummer: **0 027 593**
**B1**

(12) # EUROPÄISCHE PATENTSCHRIFT

(45) Veröffentlichungstag der Patentschrift: **25.05.83**

(21) Anmeldenummer: **80106061.7**

(22) Anmeldetag: **07.10.80**

(51) Int. Cl.³: **C 07 C 37/60,** C 07 C 39/08, C 07 C 39/17, C 07 C 39/27, C 07 C 41/26, C 07 C 43/20, C 07 C 51/367, C 07 C 65/00, C 07 C 69/00, C 07 C 79/22, C 07 C 91/44

(54) **Verfahren zur Herstellung mehrwertiger Phenole.**

(30) Priorität: **19.10.79 DE 2942366**

(43) Veröffentlichungstag der Anmeldung: **29.04.81 Patentblatt 81/17**

(45) Bekanntmachung des Hinweises auf die Patenterteilung: **25.05.83 Patentblatt 83/21**

(84) Benannte Vertragsstaaten: **BE CH DE FR GB IT LI NL**

(73) Patentinhaber: **BAYER AG, Zentralbereich Patente, Marken und Lizenzen, D-5090 Leverkusen 1, Bayerwerk (DE)**

(72) Erfinder: **Jupe, Christoph, Dr., Franz-Peter-Kürten-Weg 2, D-5000 Köln 80 (DE)**
Erfinder: **Waldmann, Helmut, Dr., Carl-Rumpff-Strasse 59, D-5090 Leverkusen (DE)**
Erfinder: **Seifert, Hermann, Dr., Ruwergasse 4, D-5000 Köln 80 (DE)**

(56) Entgegenhaltungen:

**FR-A-2 071 464**
**FR-A-2 182 668**
**FR-A-2 190 787**
**FR-A-2 201 279**
**FR-A-2 211 434**
**FR-A-2 222 344**
**FR-A-2 263 217**
**FR-A-2 318 851**
**FR-A-2 336 364**
**US-A-3 652 597**

**CHEMICAL ABSTRACTS, Band 88, Nr. 11, 13. März 1978, Seite 470, Zusammenfassung Nr. 74186d, Columbus, Ohio, US**
**CHEMICAL ABSTRACTS, Band 88, Nr. 17, 24. April 1978, Seite 509, Zusammenfassung Nr. 120774u, Columbus, Ohio, US**
**CHEMICAL ABSTRACTS, Band 87, Nr. 17, 24. Oktover 1977, Seite 674, Zusammenfassung Nr. 134590x, Columbus, Ohio, US**

EP 0 027 593 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Verfahren zur Herstellung mehrwertiger Phenole

Die vorliegende Erfindung betrifft ein verbessertes Verfahren zur Herstellung mehrwertiger Phenole durch Hydroxylierung von Phenolen.

Bei der Hydroxylierung von Phenolen werden eine oder mehrere Hydroxylgruppen in Phenole eingeführt, wobei aromatische Verbindungen entstehen, die zwei oder mehr direkt an einen oder mehrere aromatische Kerne gebundene Hydroxylgruppen besitzen (= mehrwertige Phenole). Die wichtigsten mehrwertigen Phenole sind solche, die zwei Hydroxylgruppen an einem aromatischen Kern besitzen und sich von Phenol, Naphthalin, Anthracen und Phenanthren ableiten. Diese zweiwertigen Phenole stellen technisch wichtige Verbindungen dar, die in großen Mengen hergestellt werden und beispielsweise auf dem Gebiet der Photographie, der Farb- und Kunststoffe, der Geruchs- und Geschmacksstoffe, insbesondere als Zwischenprodukte auf diesen Gebieten verwendet werden (siehe z. B. Kirk-Othmer, Encyclopedia of Chemical Technology, Second Edition, Vol. 11, Seiten 462 bis 492, insbesondere Seiten 469 und 488 [1966]).

Es sind eine Reihe von Verfahren zur Herstellung von mehrwertigen Phenolen bekannt, bei denen mehrwertige Phenole durch Hydroxylierung von Phenolen erhalten werden. Als Hydroxylierungsmittel werden dabei häufig peroxidische Verbindungen verwendet, beispielsweise Wasserstoffperoxid, Peroxosalze oder Persäuren, insbesondere Percarbonsäuren (siehe z. B. DE-AS 2 064 497, DE-AS 1 593 968, DE-AS 1 543 830, DE-AS 2 364 181, DE-AS 2 407 398, DE-OS 2 658 866, DE-OS 2 658 943, JP-OS 54-55 [1979] und JP-OS 54-66 629 [1979]).

Bei diesen Verfahren werden im allgemeinen die zu hydroxylierenden Phenole in großem Überschuß, bezogen auf das peroxidische Hydroxylierungsmittel, eingesetzt, d. h. die zu hydroxylierenden Phenole werden nur zum Teil hydroxyliert. So wird z. B. in der DE-OS 1 593 968 beschrieben, daß ein Umwandlungsgrad des eingesetzten Phenols von 30% nicht überschritten werden soll, da sonst die Ausbeute an mehrwertigen Phenolen, in diesem Fall Diphenolen, sehr rasch abnimmt. In der DE-OS 2 364 181 wird beschrieben, daß bei der Anwendung von Persäuren als Hydroxylierungsmittel für Phenole zweckmäßigerweise nicht mehr als 0,5 Mol Persäure pro Mol Phenol eingesetzt wird, da größere Mengen an diesem Oxidationsmittel eine sekundäre Oxidation bewirken, welche die Ausbeute des gewünschten Produktes in extremem Ausmaß vermindert.

Bei der technischen Durchführung solcher Verfahren werden deshalb nach der Hydroxylierungsreaktion die nicht umgesetzten Anteile der eingesetzten Phenole zurückgewonnen und wieder in die Hydroxylierungsreaktion eingesetzt. Gegebenenfalls werden den rückgeführten Phenolen so viel frische Phenole zugefügt, wie in der Hydroxylierungsreaktion umgesetzt worden sind.

Aus der DE-OS 2 364 181 ist weiterhin bekannt, daß man Diphenole in verbesserten Ausbeuten und Selektivitäten erhält, wenn man die Umsetzung von Monophenolen in Gegenwart bestimmter Persäurestabilisatoren oder Katalysatoren durchführt, die eine Struktur aufweisen können, aufgrund der theoretisch angenommen werden kann, daß sie mit Schwermetallionen Chelatkomplexe bilden können. Bei diesem Verfahren werden, bezogen auf Monophenol, 0,05 bis 1 Gew.-% Persäurestabilisator bzw. Katalysator benötigt. In der DE-OS 2 364 181 ist angegeben, daß beim Einsatz geringerer Mengen von Persäurestabilisator bzw. Katalysator kein gewünschter Effekt erzielt wird.

Es wurde nun ein Verfahren zur Herstellung mehrwertiger Phenole durch Hydroxylierung von Phenolen mit einer weitgehend wasser- und wasserstoffperoxidfreien Lösung einer Percarbonsäure in einem organischen Lösungsmittel, wobei man unumgesetzte Phenole abtrennt und zurückführt, gefunden, das dadurch gekennzeichnet ist, daß man die Hydroxylierung unter Zusatz von 0,00005 bis 0,03 Gew.-% (bezogen auf eingesetzte Phenole) einer oder mehrerer Verbindungen aus der Gruppe Iminodiessigsäure, Nitrilotriessigsäure, Nitrilotripropionsäure, Ethylendiamintetraessigsäure, o-Phenylendiamindiessigsäure, o-Phenylendiamintetraessigsäure, 1,2-Cyclohexandiamintetraessigsäure, Dipicolinsäure, Diethylentriaminpentaessigsäure, deren Salze, deren Ester, Natriumpyrophosphat, Phosphorsäurederivate der Zusammensetzung $Na_5R_5(P_3O_{10})_2$ (mit R = 2-Ethylhexyl), Oxyethandiphosphonsäure, deren Salze und deren Ester, durchführt.

Besonders bevorzugt setzt man Nitrilotriessigsäure, Ethylendiamintetraessigsäure und/oder deren Alkali- und/oder Erdalkalisalze und/oder deren Ester ein. Innerhalb dieser Gruppe von Substanzen ist das Dinatriumsalz der Ethylendiamintetraessigsäure nochmals bevorzugt.

Im allgemeinen ist es völlig ausreichend, wenn beim erfindungsgemäßen Verfahren eine Substanz aus einer der vorstehenden Gruppen verwendet wird. Man kann aber auch mehrere solcher Substanzen in das erfindungsgemäße Verfahren einsetzen.

Die erfindungsgemäß zuzusetzende Menge der Substanz oder der Substanzen aus der vorstehenden Gruppe beträgt 0,00005 bis 0,03 Gew.-%, das sind 0,5 bis 300 ppm, jeweils bezogen auf eingesetzte Phenole. Bevorzugt beträgt diese Menge 5 bis 200 ppm, besonders bevorzugt 10 bis 100 ppm, jeweils bezogen auf eingesetzte Phenole.

Die Substanzen aus der vorstehenden Gruppe können in reiner Form der Hydroxylierungsreaktion zugesetzt werden. Hierbei ist dann für eine gute Durchmischung zu sorgen. Vorzugsweise setzt man diese Substanzen in gelöster Form der

Hydroxylierungsreaktion zu, was die Dosierung und Verteilung erleichtert. Geeignete Lösungsmittel sind beispielsweise Wasser und solche, die in der Hydroxylierungsreaktion ohnehin angewendet werden, sofern diese ein ausreichendes Lösevermögen für die Substanzen aus der vorstehenden Gruppe haben.

Die erfindungsgemäß zuzusetzenden Substanzen können an verschiedenen Stellen des Hydroxylierungsverfahrens zugesetzt werden. Beispielsweise kann der Zusatz erfolgen zu frisch in die Hydroxylierungsreaktion eingesetzten Phenolen (Frischphenolen), zu Phenolen, die in der Hydroxylierungsreaktion nicht umgesetzt, abgetrennt und rückgeführt werden (Rückphenolen) und/oder zu dem im allgemeinen aus Frisch- und Rückphenolen bestehenden Einsatzstrom in die Hydroxylierungsreaktion. Falls die Frischphenole und/oder die Rückphenole vor dem Einsatz in die Hydroxylierungsreaktion noch einer Reinigung oder sonstigen Behandlung unterworfen werden, werden die erfindungsgemäßen Zusätze zweckmäßig nach einer derartigen Reinigung oder Behandlung zugefügt. Vorzugsweise werden die Substanzen aus der vorstehenden Gruppe zugegeben, bevor die Percarbonsäure eingebracht wird.

Gemäß dem erfindungsgemäßen Verfahren können die verschiedensten Phenole hydroxyliert werden. Es kommen hierfür aromatische Verbindungen in Frage, die eine oder mehrere an einen oder mehrere aromatische Kerne gebundene Hydroxylgruppen enthalten und in die eine oder mehrere Hydroxylgruppen mit peroxidischen Hydroxylierungsmitteln eingeführt werden können.

Die Phenole können außer einer oder mehreren Hydroxylgruppen keine weiteren Substituenten aufweisen, sie können jedoch auch verschiedene Substituenten tragen, soweit diese nicht die Hydroxylierungsreaktion verhindern. Als Phenole kommen beispielsweise aromatische Hydroxyverbindungen in Frage, die sich von Benzol, Naphthalin, Phenanthren oder Anthracen ableiten und die noch mindestens ein freies Wasserstoffatom an einem aromatischen Kern aufweisen. Bei Phenolen, die sich von Benzol ableiten, befindet sich ein freies Wasserstoffatom bevorzugt in 2- oder 4-Stellung zu einer bereits vorhandenen Hydroxylgruppe. Die Phenole können außer einer oder mehreren Hydroxylgruppen an dem oder den aromatischen Kernen als Substituenten beispielsweise einen oder mehrere, gleiche oder verschiedene aliphatische, cycloaliphatische Phenyl- oder Naphthylreste enthalten. Als aliphatische Reste kommen beispielsweise geradkettige und verzweigte Reste mit beispielsweise 1 bis 10 C-Atomen in Frage, wie Methyl, Ethyl, Isopropyl, n-Butyl-i-Butyl, tert.-Butyl, n-Pentyl, 2-Methylbutyl, 3-Methylbutyl, 2-Ethylpropyl, n-Hexyl, 2-Methylpentyl, 3-Methylpentyl, 4-Methylpentyl, n-Heptyl, n-Octyl, 2-Ethylhexyl, n-Nonyl, 2-Ethylheptyl und n-Decyl. Als cycloaliphatische Reste kommen beispielsweise solche mit 3 bis 12 C-Atomen in Frage, wie Cyclopropyl, Cyclobutyl, Cyclopentyl, Cyclohexyl, Cycloheptyl, Cyclooctyl, Cyclononyl und Cyclodecyl.

In den aliphatischen, cycloaliphatischen Phenyl- und Naphthylresten können jeweils ein oder mehrere Wasserstoffatome durch Gruppen ersetzt sein, die unter den Bedingungen der Hydroxylierungsreaktion stabil sind.

Beispiele für derartige Gruppen sind eines oder mehrere Fluor-, Chlor- und/oder Bromatome; eine oder mehrere $C_1$- bis $C_5$-Alkoxy-, $C_1$- bis $C_5$-Dialkylamino-, Carboxyl-, Nitro-, Cyan-, Sulfonsäure- und/oder Carboalkoxygruppen, deren Alkoxyreste 1 bis 10 C-Atome aufweisen.

Auch der oder die aromatischen Kerne der Phenole können durch eine oder mehrere dieser Atome bzw. Gruppen substituiert sein.

Bevorzugt werden in das erfindungsgemäße Verfahren Phenole eingeführt, die sich vom Benzol ableiten.

Beispielsweise können in das erfindungsgemäße Verfahren eingesetzt werden Phenole der Formel

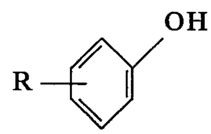

(I)

worin

R für Wasserstoff, $C_1$- bis $C_{10}$-Alkyl, $C_3$- bis $C_{12}$-Cycloalkyl, Phenyl, Naphthyl, Fluor, Chlor, Brom, eine Nitro-, Cyan-, Sulfonsäure-, Carboxyl-, Carbo-$C_1$- bis $C_{10}$-Alkoxy-, $C_1$- bis $C_3$-Alkoxy- oder $C_1$- bis $C_4$-Dialkylamino-Gruppe seht, wobei die Alkyl- und Cycloalkyl-Reste durch Fluor-, Chlor-, Bromatome, $C_1$- bis $C_5$-Alkoxy-, $C_1$- bis $C_4$-Dialkylamino-, Carboxyl-, Nitro-, Cyan-, Sulfonsäure- oder $C_1$- bis $C_{10}$-Carbalkoxy-Gruppen und die Phenyl- und Naphthyl-Reste durch Fluor, Chlor, Brom, $C_1$- bis $C_{10}$-Alkyl, $C_3$- bis $C_{12}$-Cycloalkyl, sowie Nitro-, Carboxyl-, Carbo-$C_1$- bis $C_{10}$-alkoxy-, $C_1$- bis $C_5$-Alkoxy-, Cyan-, Sulfonsäure- oder $C_1$- bis $C_4$-Dialkylamino-Gruppen substituiert sein können.

Eine bevorzugte Gruppe von Phenolen innerhalb der Formel (I) entspricht den Phenolen der Formel

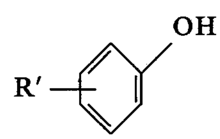

(II)

worin

R' für Wasserstoff, $C_1$- bis $C_3$-Alkyl, $C_5$- bis $C_7$-Cycloalkyl, Phenyl, Fluor, Chlor, eine Nitro-, Sulfonsäure-, Carboxyl-, Carbo-$C_1$-bis $C_3$-alkoxy-, $C_1$- bis $C_2$-Alkoxy- oder $C_1$- bis $C_2$-Dialkylamino-Gruppe steht.

Besonders bevorzugt setzt man in das erfindungsgemäße Verfahren einwertige Phenole der Formel

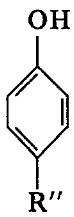

(III)

ein,

worin

R'' für Wasserstoff, $C_1$- bis $C_5$-Alkyl, $C_5$- bis $C_7$-Cycloalkyl, Phenyl, Fluor, Chlor, eine Nitro-, Sulfonsäure-, Carboxyl-, Carbo-$C_1$- bis $C_3$-alkoxy-, $C_1$- bis $C_3$-Alkoxy- oder $C_1$- bis $C_2$-Dialkylamino-Gruppe bedeutet.

Im einzelnen seien als Phenole, die zum Einsatz in das erfindungsgemäße Verfahren geeignet sind, beispielsweise genannt: Phenol, o-Kresol, m-Kresol, p-Kresol, p-Cyclohexylphenol, o-Cyclohexylphenol, p-Phenylphenol, o-Phenylphenol, m-Phenylphenol, p-Ethylphenol, o-Ethylphenol, m-Ethylphenol, o-Isopropylphenol, p-Isopropylphenol, o- und p-tert.-Butylphenol, p-Nitrophenol, o-Nitrophenol, m-Nitrophenol, 2-Brom-4-methylphenol, p-Chlorphenol, o-Chlorphenol, m-Chlorphenol, p-Carbomethoxyphenol, Salicylsäureethylester, p-Cyclopentylphenol, o-Dimethylaminophenol, p-Cyano-phenol, p-Methoxy-phenol, o-Isopropoxyphenol, p-Ethoxy-phenol, 3,5-Diethyl-phenol, Thymol, Phenol, Carvacrol, 1,2,3-Xylenol, 1,2,4-Xylenol, 1,3,2-Xylenol, 1,3,4-Xylenol, 1,3,5-Xylenol, 1,4,2-Xylenol, $\alpha$-Naphthol, $\beta$-Naphthol, 1-Hydroxy-4-methylnaphthalin, 1-Hydroxy-2-methylnaphthalin, 2-Hydroxy-1-methylnaphthalin, 2-Hydroxy-6-methylnaphthalin, 1-Hydroxy-4-isopropylnaphthalin, 1-Hydroxy-4-t-butylnaphthalin, 1-Hydroxy-6-phenylnaphthalin, 1-Hydroxy-6-methoxynaphthalin, 1-Hydroxy-anthracen, 2-Hydroxy-anthracen.

Ganz besonders bevorzugt setzt man in das erfindungsgemäße Verfahren Phenol ($C_6H_5OH$) ein.

Das erfindungsgemäße Verfahren ist für die Herstellung mehrwertiger Phenole durch Hydroxylierung von Phenolen mit einer weitgehend wasser- und wasserstoffperoxidfreien Lösung einer Percarbonsäure geeignet. Außer dem Zusatz von Substanzen aus der vorstehend genannten Gruppe kann diese Hydroxylierung in an sich bekannter Weise durchgeführt werden (s. DE-OS 2 658 943). Die Percarbonsäure enthält dabei vorzugsweise 1 bis 4 C-Atome und das organische Lösungsmittel ist dabei beispielsweise Benzol, 1,2-Dichlorpropan oder Essigsäureethylester.

Das erfindungsgemäße Verfahren hat den Vorteil, daß sich mehrwertige Phenole durch Hydroxylierung von Phenolen mit Percarbonsäuren mit sehr guten Selektivitäten herstellen lassen. Die Selektivitäten sind in den meisten Fällen besser als in dem Verfahren gemäß der DE-OS 2 364 181. Ein weiterer Vorteil des erfindungsgemäßen Verfahrens besteht darin, daß diese guten Selektivitäten mit Zusätzen von geringeren Mengen an Hilfsstoffen erzielt werden als in dem Verfahren der DE-OS 2 364 181.

Es ist als ausgesprochen überraschend anzusehen, daß das erfindungsgemäße Verfahren so gute Ergebnisse liefert, denn aufgrund der Angaben in der DE-OS 2 364 181 konnte nicht erwartet werden, daß Zusätze von Hilfsstoffen der beschriebenen Art in Mengen von weniger als 0,05 Gew.-% (bezogen auf eingesetzte Phenole) vorteilhafte Wirkungen haben.

## Beispiele

### Beispiel 1

20 kg Phenol (Handelsware) und 150 kg Phenol, das aus der Aufarbeitung von bereits mit Perpropionsäure umgesetztem Phenol stammte, wurden unter Stickstoff bei 60° C zusammengegeben und mit 200 g einer 3gew.-%igen Lösung des Dihydrats des Dinatriumsalzes der Ethylendiamintetraessigsäure in Wasser versetzt und gut durchmischt. Das so vorbereitete Phenolgemisch wurde mit einer Durchsatzgeschwindigkeit von 10 kg pro Stunde dem ersten Reaktionsgefäß einer vierstufigen gerühten Kesselkaskade zugeführt, die auf 40° C thermostatisiert wurde. Gleichzeitig wurden dem ersten Reaktionsgefäß 2,4 kg pro Sunde einer 20gew.-%igen Lösung von Perpropionsäure in einem Benzol-Propionsäure-Gemisch (5 Gew.-Teile Benzol auf 1 Gew.-Teil Propionsäure) zugeführt. Die Reaktionsgefäße wiesen ein nutzbares Volumen von 1,7 l auf. Durch Fluten der Gefäße, Stickstoffüberlagerung auf dem ersten Kessel und entsprechende Überläufe wurde das Reaktionsgemisch einmal durch alle Gefäße gedrückt.

Das Reaktionsgemisch wies hinter dem letzten Kessel einen Restgehalt von 0,03 Gew.-% Perpropionsäure auf, entsprechend einem Umsatz von 99,2%. Der Gehalt an Brenzkatechin im Reaktionsgemisch betrug 2,6 Gew.-%, der Gehalt an Hydrochinon 1,4 Gew.-%, entsprechend Selektivitäten von 55% für Brenzkatechin und 30% für Hydrochinon, jeweils bezogen auf umgesetzte Perpropionsäure.

### Beispiel 2
(Vergleichsbeispiel,
Arbeitsweise gemäß DE-OS 2 364 181)

Bei einer Wiederholung des Beispiels 1, bei der 0,5 Gew.-% des Dihydrates des Dinatriumsalzes der Ethylendiamintetraessigsäure, bezogen auf Phenol, zugesetzt wurden, betrug der Umsatz der Perpropionsäure 98,9%, der Gehalt

im Reaktionsgemisch an Brenzkatechin 2,27 Gew.-% und der Gehalt an Hydrochinon 1,31 Gew.-%, entsprechend Selektivitäten von 48% für Brenzkatechin und 28% für Hydrochinon, jeweils bezogen auf umgesetzte Perpropionsäure.

Beispiel 3

Die Schmelze von 94,1 g Phenol, welches aus der destillativen Aufarbeitung von bereits mit einem Überschuß an Perpropionsäure umgesetzten Phenol stammte, wurde mit 0,1 ml einer Lösung von 4 g Oxyethandiphosphonsäure in 96 g Wasser versetzt. Anschließend wurde mit 4,7 g Perpropionsäure bei 45°C umgesetzt. Nach 60 Minuten wurde ein Umsatz von 98% der eingesetzten Perpropionsäure gefunden; das Reaktionsgemisch enthielt 2,8 g Brenzkatechin und 1,6 g Hydrochinon, entsprechend Selektivitäten von 50% für Brenzkatechin und 28% für Hydrochinon.

**Patentansprüche**

1. Verfahren zur Herstellung mehrwertiger Phenole durch Hydroxylierung von Phenolen mit einer weitgehend Wasser- und Wasserstoffperoxid-freien Lösung einer Percarbonsäure in einem organischen Lösungsmittel, wobei man unumgesetzte Phenole abtrennt und zurückführt, dadurch gekennzeichnet, daß man die Hydroxylierung unter Zusatz von 0,00005 bis 0,03 Gew.-% bezogen auf eingesetzte Phenole) einer oder mehrerer Verbindungen aus der Gruppe Iminodiessigsäure, Nitrilotriessigsäure, Nitrilotripropionsäure, Ethylendiamintetraessigsäure, o-Phenylendiamindiessigsäure, o-Phenylendiamintetraessigsäure, 1,2-Cyclohexandiamintetraessigsäure, Dipicolonsäure, Diethylentriaminpentaessigsäure, deren Salze, deren Ester, Natriumpyrophosphat, Phosphorsäurederivate der Zusammensetzung $Na_5R_5(P_3O_{10})_2$ (mit R = 2-Ethylhexyl), Oxyethandiphosphonsäure, deren Salze und deren Ester, durchführt.

2. Verfahren nach Anspruch 1, dadurch gekennzeichnet, daß das zu hydroxylierende Phenol außer einer oder mehreren Hydroxylgruppen keine weiteren Substituenten aufweist.

3. Verfahren nach Anspruch 1, dadurch gekennzeichnet, daß das zu hydroxylierende Phenol außer einer oder mehreren Hydroxylgruppen an dem oder den aromatischen Kernen als Substituenten einen oder mehrere, gleiche oder verschiedene aliphatische, cycloaliphatische Phenyl- und/oder Naphthyl-Reste; eines oder mehrere Fluor-, Chlor- und/oder Bromatome; eine oder mehrere $C_1$- bis $C_5$-Alkoxy-, $C_1$- bis $C_5$-Dialkylamino-, Carboxyl-, Nitro-, Cyan-, Sulfonsäure- und/oder Carboalkoxygruppen, deren Alkoxyreste 1 bis 10 C-Atome aufweisen, enthält.

**Claims**

1. Process for the preparation of polyhydric phenols by hydroxylation of phenols with a solution, which is substantially free of water and hydrogen peroxide, of a percarboxylic acid in an organich solvent, non-reacted phenols being removed and recycled, characterised in that the hydroxylation is carried out with the addition of 0.00005 to 0.03% by weight (relative to the phenols employed) of one or more compounds from the group comprising iminodiacetic acid, nitrilotriacetic acid, nitrilotripropionic acid, ethylenediaminetetraacetic acid, o-phenylenediaminediacetic acid, o-phenylenediamintetraacetic acid, 1,2-cyclohexanediaminetetraacetic acid, dipicolinic acid, diethylenetriaminepentaacetic acid, salts thereof, esters thereof, sodium pyrophosphate, phosphoric acid derivatives having the composition $Na_5R_5(P_3O_{10})_2$ (in which R = 2-ethylhexyl), oxyethanediphosphonic acid, salts thereof and esters thereof.

2. Process according to Claim 1, characterised in that the phenol to be hydroxylated contains no other substituents besides one or more hydroxyl groups.

3. Process according to Claim 1, characterised in that, besides one or more hydroxyl groups, the phenol to be hydroxylated contains, as substituents, one or more identical or different aliphatic, cycloaliphatic, phenyl and/or naphthyl radicals on the aromatic nucleus or nuclei; one or more fluorine, chlorine and/or bromine atoms; one or more $C_1$- to $C_5$-alkoxy, $C_1$- to $C_5$-dialkylamino, carboxyl, nitro, cyano, sulphonic acid groups and/or carbalkoxy groups, the alkoxy radicals of which have 1 to 10 C atoms.

**Revendications**

1. Procédé de préparation de phénols polyvalents par hydroxylation de phénols avec une solution d'un acide percarboxylique dans un solvant organique, cette solution étant essentiellement exempte d'eau et de peroxyde d'hydrogène, en séparant et en recyclant les phénols n'ayant pas réagi, caractérisé en ce qu'on effectue l'hydroxylation en ajoutant 0,00005 à 0,03% en poids (calculé sur les phénols utilisés) d'un ou de plusieurs composés choisis parmi le groupe comprenant l'acide iminodiacétique, l'acide nitrilotriacétique, l'acide nitrilotripropionique, l'acide éthylène-diaminie-tétracétique, l'acide o-phénylène-diaminodiacétique, l'acide o-phénylène-diaminotétracétique, l'acide 1,2-cyclohexane-diaminotétracétique, l'acide dipicolinique, l'acide diéthylène-triaminopentacétique, leurs sels, leurs esters, le pyrophosphate de sodium, les dérivés d'acide phosphorique de la composition $Na_5R_5(P_3O_{10})_2$ (où R = groupe 2-éthylhexyle), l'acide oxyéthane-diphosphonique, leurs sels et leurs esters.

2. Procédé suivant la revendication 1, caractérisé en ce que, hormis un ou plusieurs groupes hydroxy, le phénol à hydroxyler ne comporte aucun autre substituant.

3. Procédé suivant la revendication 1, caractérisé en ce que, hormis un ou plusieurs groupes hydroxy, le phénol à hydroxyler contient, comme substituants sur le ou les noyaux aromatiques, un ou plusieurs radicaux phényle et/ou naphtyle aliphatiques ou cycloaliphatiques, identiques ou différents; un ou plusieurs atomes de fluor, de chlore et/ou de brome; un ou plusieurs groupes alcoxy en $C_1-C_5$, dialkylamino en $C_1-C_5$, carboxy, nitro, cyano, acide sulfonique et/ou carbalcoxy dont les groupes alcoxy contiennent 1 à 10 atomes de carbone.